# EUROPEAN PATENT APPLICATION

(11) **EP 2 055 778 A1**
(43) Date of publication of application: **06.05.2009**
(21) Application number: 07830130.6
(22) Date of filing: 12.10.2007
(51) Int. Cl.: C12N 15/29, A01H 1/00, A01H 5/00, C07K 14/415

(54) **MUTANT PLANT AND METHOD FOR PRODUCTION THEREOF**

(30) Priority: 12.10.2006 JP 2006278988
(71) Applicant: Riken, Wako-shi, Saitama 351-0198 (JP)
(72) Inventor: TOMINAGA, Rumi, Yokohama-shi Kanagawa 230-0045 (JP); WADA, Takuji, Yokohama-shi Kanagawa 230-0045 (JP)
(74) Representative: Denison, Christopher Marcus
(86) International application number: PCT/JP2007/070396
(87) International publication number: WO 2008/044800

(57) **Abstract**

According to the present invention, a novel mutated plant is provided based on findings obtained by analyzing the function of a functionally unknown gene having a CPC-like Myb sequence.
The function of any one of the following proteins is suppressed:
(a) a protein comprising the amino acid sequence set forth in SEQ ID NO: 2; and
(b) a protein which comprises an amino acid sequence having a substitution, deletion, addition, or insertion of one or more amino acids in the amino acid sequence set forth in SEQ ID NO:2, and is capable of acting as a transcription factor for controlling epidermal cell differentiation.

A plant having a characteristic phenotype, for example which can grow to a larger size than the wild-type plant, can be obtained by suppressing the function of the above protein.

## Description

### Technical Field

The present invention relates to a mutated plant having characteristic morphologies and a method for production thereof.

### Background Art

Non-Patent Document 1 discloses CAPRICE (hereinafter referred to as a CPC protein), which is a protein having the R3 MYB motif and is usually involved in root hair cell differentiation in *Arabidopsis thaliana.* Non-Patent Document 2 describes that cell-to-cell movement of such CPC protein (from non-root hair cells to root hair cells) takes place such that the CPC protein suppresses the expression of the homeodomain-leucine zipper gene (GLABRA2).

Four types of CPC-like Myb sequences such as the TRIPTYCHON gene (TRY gene) have been found in the genome of *Arabidopsis thaliana.* Since trichome clusters are observed in a strain lacking the TRY gene, the TRY protein controls trichome formation. Kirik et al. have already identified the two of the above four types of CPC-like Myb sequences (referred to as ETC 1 and ETC2) and genetically analyzed the relationships between such sequences and CPC or TRY (Non-Patent Documents 3 and 4). The group of David Marks analyzed the relationship between TRY and At1g01380 (ETC1) and they demonstrated that GL3 controls TRY gene expression but not ETC1 expression (Non-Patent Document 5).

However, other genes each comprising a CPC-like Myb sequence have not been analyzed thus far. Therefore, the functions of such genes remain unknown.

Non-Patent Document 1: Wada, T., Tachibana, T., Shimura, Y., and Okada, K. (1997). Epidermal cell differentiation in Arabidopsis determined by a Myb homolog, CPC. Science 277, 1113-1116.

Non-Patent Document 2: Wada, T., Kurata, T., Tominaga, R., Koshino-Kimura, Y., Tachibana, T., Goto, K., Marks, M.D., Shimura, Y., and Okada, K. (2002). Role of a positive regulator of root hair development, CAPRICE, in Arabidopsis root epidermal cell differentiation. Development 129, 5409-5419.

Non-Patent Document 3: Kirik, V., Simon, M., Huelskamp, M., and Schiefelbein, J. (2004b). The ENHANCER OF TRY AND CPC1 gene acts redundantly with TRIPTYCHON and CAPRICE in trichome and root hair cell patterning in Arabidopsis. Dev Biol 268, 506-513.

Non-Patent Document 4: Kirik, V., Simon, M., Wester, K., Schiefelbein, J., and Hulskamp, M. (2004a). ENHANCER of TRY and CPC 2 (ETC2) reveals redundancy in the region-specific control of trichome development of Arabidopsis. Plant Mol Biol 55, 389-398.

Non-Patent Document 5: Esch, J. J., Chen, M. A., Hillestad, M., and Marks, M.D. (2004). Comparison of TRY and the closely related At1g01380 gene in controlling Arabidopsis trichome patterning. Plant J 40, 860-869.

### Disclosure of the Invention

It is an objective of the present invention to provide a novel mutated plant based on findings obtained by analyzing the function of a functionally unknown gene having a CPC-like Myb sequence and to provide a method for producing the mutated plant.

As a result of analysis of the function of a functionally unknown gene having a CPC-like Myb sequence, the present inventors have found that a strain lacking such gene has characteristic morphologies compared with the wild-type plant. This has led to the completion of the present invention.

Specifically, the present invention encompasses the following.
(1) A mutated plant in which the function of any one of the following proteins (a) to (c) is suppressed:
   (a) a protein comprising the amino acid sequence set forth in SEQ ID NO: 2;
   (b) a protein which comprises an amino acid sequence having a substitution, deletion, addition, or insertion of one or more amino acids in the amino acid sequence set forth in SEQ ID NO: 2, and is capable of acting as a transcription factor for controlling epidermal cell differentiation; and
   (c) a protein which is encoded by a polynucleotide having a nucleotide sequence with a substitution, deletion, addition, or insertion of one or more nucleotides in the nucleotide sequence set forth in SEQ ID NO: 1, and is capable of acting as a transcription factor for controlling epidermal cell differentiation.
(2) The mutated plant according to (1), wherein the mutated plant lacks a gene encoding any one of the above proteins (a) to (c).
(3) The mutated plant according to (1), wherein the mutated plant is a dicotyledon.
(4) The mutated plant according to (1), wherein the mutated plant is a plant of the family *Brassicaceae.*
(5) A method for producing a mutated plant, comprising suppressing the function of any one of the following proteins (a) to (c) in a target plant:
   (a) a protein comprising the amino acid sequence set forth in SEQ ID NO: 2;
   (b) a protein which comprises an amino acid sequence having a substitution, deletion, addition, or insertion of one or more amino acids in the amino acid sequence set forth in SEQ ID NO: 2, and is capable of acting as a transcription factor for controlling epidermal cell differentiation; and
   (c) a protein which is encoded by a polynucleotide having a nucleotide sequence with a substitution, deletion, addition, or insertion of one or more nucleotides in the nucleotide sequence set forth in SEQ ID NO: 1, and is capable of acting as a transcription factor for controlling epidermal cell differentiation.
(6) The method for producing a mutated plant according to (5), comprising deleting a gene encoding any one of the above proteins (a) to (c).
(7) The method for producing a mutated plant according to (5), wherein the target plant is a dicotyledon.
(8) The method for producing a mutated plant according to (5), wherein the target plant is a plant of the family *Brassicaceae.*
(9) A method for producing a mutated plant which grows to a larger size or flowers earlier than the wild-type plant, comprising suppressing the function of any one of the following proteins (a) to (c) in a target plant:
   (a) a protein comprising the amino acid sequence set forth in SEQ ID NO: 2;
   (b) a protein which comprises an amino acid sequence having a substitution, deletion, addition, or insertion of one or more amino acids in the amino acid sequence set forth in SEQ ID NO: 2, and is capable of acting as a transcription factor for controlling epidermal cell differentiation; and
   (c) a protein which is encoded by a polynucleotide having a nucleotide sequence with a substitution, deletion, addition, or insertion of one or more nucleotides in the nucleotide sequence set forth in SEQ ID NO: 1, and is capable of acting as a transcription factor for controlling epidermal cell differentiation.
(10) The method for producing a mutated plant according to (9), comprising deleting a gene encoding any one of the above proteins (a) to (c).
(11) The method for producing a mutated plant according to (9), wherein the target plant is a dicotyledon.
(12) The method for producing a mutated plant according to (9), wherein the target plant is a plant of the family *Brassicaceae.*

This description includes part or all of the contents as disclosed in the description of Japanese Patent Application No. 2006-278988, which is a priority document of the present application.

### Brief Description of the Drawings

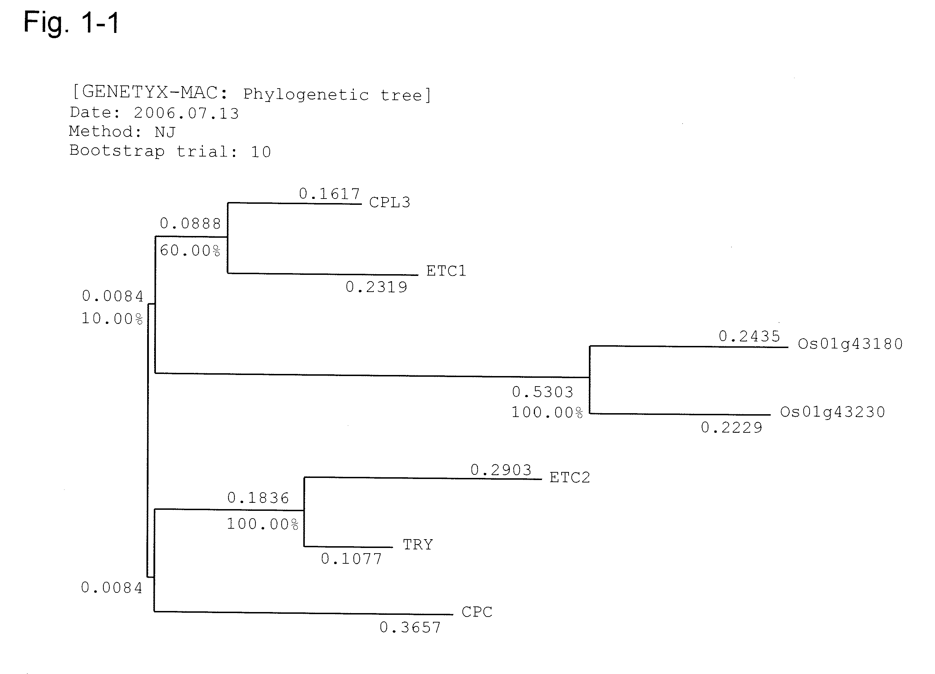

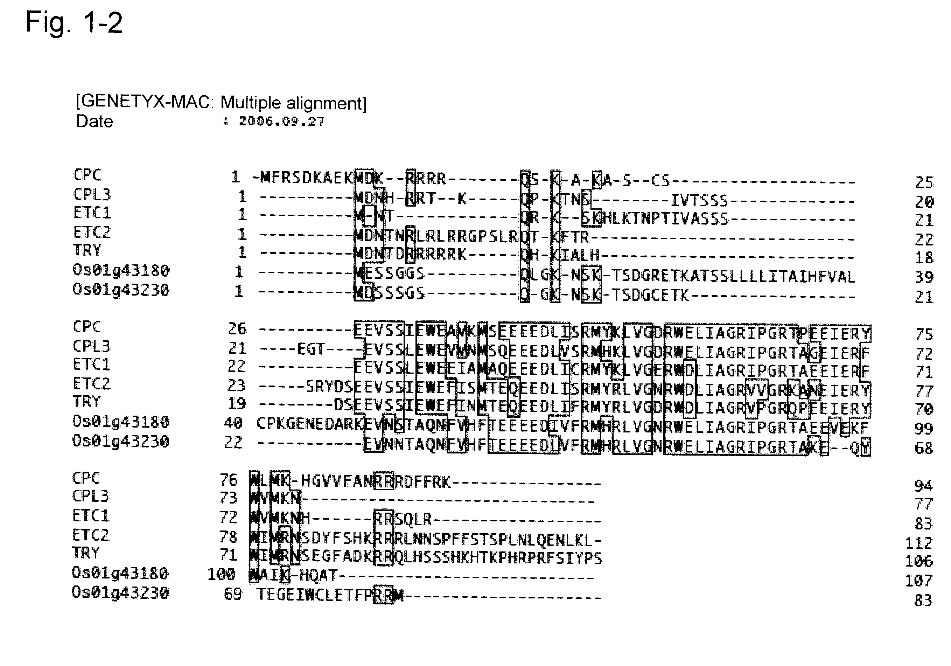

Fig. 1-1 is a phylogenetic tree showing the relationship between the CPL3 gene and rice-derived orthologs.
Fig. 1-2 shows the alignment of the amino acid sequences of LOC_Os01g43180, LOC_Os01g43230, CPC, TRY, ETC1, ETC2, and CPL3.
Fig. 2 shows the alignment of the amino acid sequences of the CPC protein, the ETC1 protein, the ETC2 protein, the TRY protein, and the CPL3 protein, and schematically shows the T-DNA insertion sites of mutants each lacking the relevant gene.
Fig. 3 is a characteristic graph showing the results of determining the root hair number in double mutants and triple mutants with respect to the CPC gene, the TYR gene, the ETC1 gene, the ETC2 gene, and the CPL3 gene.
Fig. 4 is a characteristic graph showing the results of determining the trichome number in double mutants and triple mutants with respect to the CPC gene, the TYR gene, the ETC1 gene, the ETC2 gene, and the CPL3 gene.
Fig. 5 is a characteristic graph showing the results of measuring the raw weight in the cpl3-1 mutant, the 35S::CPL3 transformant, the CPL3::CPL3 transformant, and the wild-type plant, at the same growth stage.
Fig. 6 shows images of the cpl3-1 mutant, the 35S::CPL3 transformant, the CPL3::CPL3 transformant, and the wild-type plant, at the same growth stage.
Fig. 7 is a characteristic graph showing the results of measuring the endoreduplication level in the cpl3-1 mutant, the CPL3::CPL3 transformant, and the wild-type plant.
Fig. 8 is a characteristic graph showing the results of determining the true leaf number in each of mutants and transformants.
Fig. 9 is a characteristic graph showing the results of determining the number of days until bolting of individual mutants and transformants.
Fig. 10 shows images indicating the results of observing the cell phenotypes in leaves and hypocotyls of the cpl3-1 mutant, the CPL3::CPL3 transformant, and the wild-type plant, with the use of an optical microscope.
Fig. 11 is a characteristic graph showing the results of comparing the root hair numbers among transformants each overexpressing the CPC gene, the ETC1 gene, the ETC2 gene, the TRY gene, or the CPL3 gene under the control of the 35S promoter or its own promoter.
Fig. 12 is a characteristic graph showing the results of comparing the trichome numbers among transformants each overexpressing the CPC gene, the ETC1 gene, the ETC2 gene, the TRY gene, or the CPL3 gene under the control of the 35S promoter or its own promoter.
Fig. 13 shows images indicating the results of examining the gene expression pattern with the use of transformants expressing the GUS protein under the control of the promoter of the CPC gene, the ETC1 gene, the ETC2 gene, the TRY gene, or the CPL3 gene.
Fig. 14 shows the results of examining the expression pattern of each of the CPC gene, the ETC 1 gene, the ETC2 gene, the TRY gene, and the CPL3 gene with the use of transformants highly expressing the GFP fusion protein, and the results of examining the expression pattern of the respective gene via RT-PCR and in *situ* hybridization.
Fig. 15 shows real-time PCR results indicating the CO, FT, SOC1, and CPL3 expression levels in cpc, try, etc1, etc2, and cpl3 mutated plants.

### Best Mode for Carrying Out the Invention

Hereinafter, the present invention will be described in greater detail.

The mutated plant of the present invention is obtained by deleting the function of a specific protein found in the wild-type plant. The mutated plant of the present invention is characterized in that it grows to a larger size than the wild-type plant. Specifically, the mutated plant of the present invention is characterized in that its weight (measured when cultivated to attain a size comparable to that of the wild-type plant under the same conditions) is significantly greater than that of the wild-type plant. In addition, the mutated plant of the present invention is characterized in that it flowers earlier than the wild-type plant.

The phrase "deleting the function of a protein" used herein is intended to include deletion of a gene encoding the protein from the genome, inhibition of the expression of a gene encoding the protein, and reduction of the activity of the protein.

More specifically, examples of a method for deleting a gene encoding particular protein that can be used include, but are not limited to, a method involving homologous recombination and a method using a transposon(s). In addition, upon deletion of the gene, the full-length sequence or a partial sequence of the gene may be deleted.

Further, examples of a method for inhibiting the expression of a gene encoding a particular protein that can be used include, but are not limited to, a method for deleting a promoter that controls the expression of the gene, a method for substituting a promoter that controls the expression of the gene with an expression-inducing promoter, a method for mutating a promoter that controls the expression of the gene, a method for degrading transcription products of the gene via RNA interference, and a method for inhibiting translation of the gene with the use of antisense RNA.

Furthermore, an example of a method for reducing the activity of a particular protein that can be used includes a method which comprises making a substance having a function to specifically bind to the protein and to suppress its activity to be acted on the protein. Examples of such substance that can be used include an antibody and a suppressor capable of inhibiting the function of the protein.

In the mutated plant of the present invention, the function of the protein encoded by the gene registered as At4g01060 in *Arabidopsis thaliana* is deleted. Specifically, the gene registered as At4g01060 is herein referred to as the CPL3 gene, which encodes a protein having a CPC-like Myb sequence. The nucleotide sequence of the CPL3 gene is depicted in SEQ ID NO: 1. The amino acid sequence of the CPL3 protein encoded by the CPL3 gene is depicted in SEQ ID NO: 2.

Thus, the mutated plant of the present invention lacks the function of the following protein (1), (2) or (3):
(1) a protein comprising the amino acid sequence set forth in SEQ ID NO: 2;
(2) a protein which comprises an amino acid sequence having a substitution, deletion, addition, or insertion of one or more amino acids in the amino acid sequence set forth in SEQ ID NO: 2, and is capable of acting as a transcription factor for controlling epidermal cell differentiation; or
(3) a protein which is encoded by a polynucleotide having a nucleotide sequence with a substitution, deletion, addition, or insertion of one or more nucleotides in the nucleotide sequence set forth in SEQ ID NO: 1, and is capable of acting as a transcription factor that controls epidermal cell differentiation.

The expression "more amino acids" used herein means, for example, 2 to 33 amino acid residues, preferably 2 to 15 amino acid residues, and more preferably 2 to 5 amino acid residues. In addition, examples of a positions into which a substitution, deletion, addition, or insertion of one or more amino acids is introduced include regions of positions 3 to 5 and 21 to 32, and the position 77 and the following regions in the amino acid sequence set forth in SEQ ID NO: 2. Since these regions are non-CPC-like Myb sequence regions, the function of the CPL3 protein would not be significantly affected by introducing a substitution, deletion, addition, or insertion of one or more amino acids into said regions.

In addition, the expression "more nucleotides" means, as derived from the above expression "more amino acids," for example, 6 to 99 nucleotides, preferably 6 to 45 nucleotides, and more preferably 6 to 15 nucleotides. Also, examples of a position into which a substitution, deletion, addition, or insertion of one or more nucleotides is introduced include, as derived from the above "more amino acids," the regions of the positions 9 to 15 and 63 to 96, and the position 231 and the following regions in the nucleotide sequence set forth in SEQ ID NO: 1.

Further, the present invention can be applied to any plant. Thus, the present invention can be applied to any plant as long as the wild-type plant contains a protein defined as above. For instance, the present invention is applied particularly preferably to a dicotyledon and most preferably to a plant of the family *Brassicaceae* represented by *Arabidopsis thaliana.*

Examples of a plant of the family *Brassicaceae* that can be used include plants belonging to the genus *Brassica* such as napa cabbage (*Hakusai*) (*B. campestris*), Japanese mustard spinach (*Komatsuna*) (*B. campestris var. peruviridis*), *Karashina* (*B. juncea*), *Takana* (*B. juncea var. integlifolia*), *Hagoromo kanran* (kale) (*B. oleracea var. acephala*), *Habotan* (*B. oleracea var. acephala*), cauliflower (*B. oleracea var. botrytis*), cabbage (*B. oleracea var. capitata*), Brussels sprouts (*B. oleracea var. gemmifera*), kohlrabi (*B. oleracea var. gongylodes*), broccoli (*B. oleracea var. italica*), turnip *(B. rapa*), Bok Choy (*B. rapa var. chinensis*), turnip green (*B. rapa var. hakabura*), *Mizuna (B. rapa var. lancinifolia*), and *Brassica rapa var. nippo-oleifera* (*B. rapa var. nippo-oleifera*). In addition, examples of a plant of the family *Brassicaceae* that can be used include plants belonging to the genus *Nasturtium* such as Cresson (watercress) *(N. officinale*); plants belonging to the genus *Lepidium* such as Garden cress (cress) (*L. sativum);* plants belonging to the genus *Raphanus* such as seeding radish (*R. sativus*) and radish (*R. sativus var. radicula*); plants belonging to the genus *Eruca* such as Rocket (*Rucola*) (*E. vesicaria*); plants belonging to the genus *Wasabia*/*Eutrema* such as Japanese horseradish (*W. japonica*); plants belonging to the genus *Armoracia* such as horseradish (*A. rusticana*); plants belonging to the genus *Matthiola* such as *Mathiola incana* (stock) (*M. incana*); plants belonging to the genus *Capsella* such as shepherd's purse (C. *bursa-pastoris*); plants belonging to the genus *Arabidopsis* such as *Arabidopsis thaliana* (Thale Cress or Mouse-ear Cress); plants belonging to the genus *Orychophragmus* such as *Orychophragmus violaceus* (*Shokatsusai* or *Murasakihanana*); and plants belonging to the genus *Rorippa.*

Meanwhile, the mutated plant of the present invention is not limited to the above dicotyledons and it may be a monocotyledon represented by rice. For instance, it is possible to identify a gene corresponding to the CPL3 gene in the rice genome by searching for such gene in a database containing the rice genome sequence data with the use of the amino acid sequence set forth in SEQ ID NO: 2 as a search key. Specifically, it was possible to identify two orthologs of the CPL3 gene in the rice genome database. These two ortholog genes each existing on the chromosome 1 have been registered as LOC_Os01g43180 and LOC_Os01g43230. The CPL3 gene has 63% homology at the amino acid level with LOC_Os01g43180 and 59% homology at the amino acid level with LOC_Os01g43230. The amino acid sequence of the protein encoded by LOC_Os01g43180 is depicted in SEQ ID NO: 3. The amino acid sequence of the protein encoded by LOC_Os01g43230 is depicted in SEQ ID NO: 4.

In addition, regarding LOC_Os01g43180 and LOC_Os01g43230, homologies with the TRY gene, the CPC gene, the ETC1 gene, and the ETC2 gene were calculated. LOC_Os01g43180 has 61% homology at the amino acid level with CPC, 54% homology at the amino acid level with ETC2, 53% homology at the amino acid level with ETC1, and 51% homology at the amino acid level with TRY. LOC_Os01g43230 has 56% homology at the amino acid level with ETC2, 54% homology at the amino acid level with CPC, 49% homology at the amino acid level with ETC1, and 44% homology at the amino acid level with TRY. Fig. 1-1 is a phylogenetic tree showing the above results. Further, fig. 1-2 shows the results of alignment of the amino acid sequences of LOC_Os01g43180, LOC_Os01g43230, CPC, TRY, ETC1, ETC2, and CPL3.

As described above, LOC_Os01g43180 and LOC_Os01g43230 found in the rice genome have the highest homology with CPL3 among proteins each comprising a CPC-like Myb sequence. Therefore, it can be judged that LOC_Os01g43180 and LOC_Os01g43230 have a high probability of having functions equivalent to those of the CPL3 gene of *Arabidopsis thaliana.*

The mutated plant of the present invention as defined above is characterized in that it grows to a larger size than the wild-type plant. Thus, in a case in which a target plant for mutation is edible, productivity improvement can be expected as a result of size enlargement according to the present invention. In addition, in a case in which a substance is produced in a target plant for mutation, it is possible to improve the productivity of such useful substance according to the present invention. Herein, such substance may be a substance originally produced by the wild-type plant or a substance produced via transformation of the wild-type plant.

Further, the mutated plant of the present invention is characterized in that it flowers earlier than the wild-type plant. Therefore, fruit or seed formation can be promoted and thus the mutated plant of the present invention is advantageous in that it is possible to harvest a crop that can usually be harvested just once a year a plurality of times a year. Moreover, such characteristics of the mutated plant of the present invention enable to save time and cost for crop harvesting.

The present invention is hereafter described in greater detail with reference to the following examples, although the scope of the present invention is not limited thereto.

### (Experimental example 1) Screening for a mutated plant lacking the CPL3 gene

In this Example, the *Arabidopsis thaliana* ecotype Col-0 (hereinafter referred to as the "wild-type *Arabidopsis thaliana"*) was used as the wild-type plant. In this Example, a cpl3-1 mutant in which a gene comprising the nucleotide sequence set forth in SEQ ID NO: 1 had been mutated was screened form the Wisconsin T-DNA group.

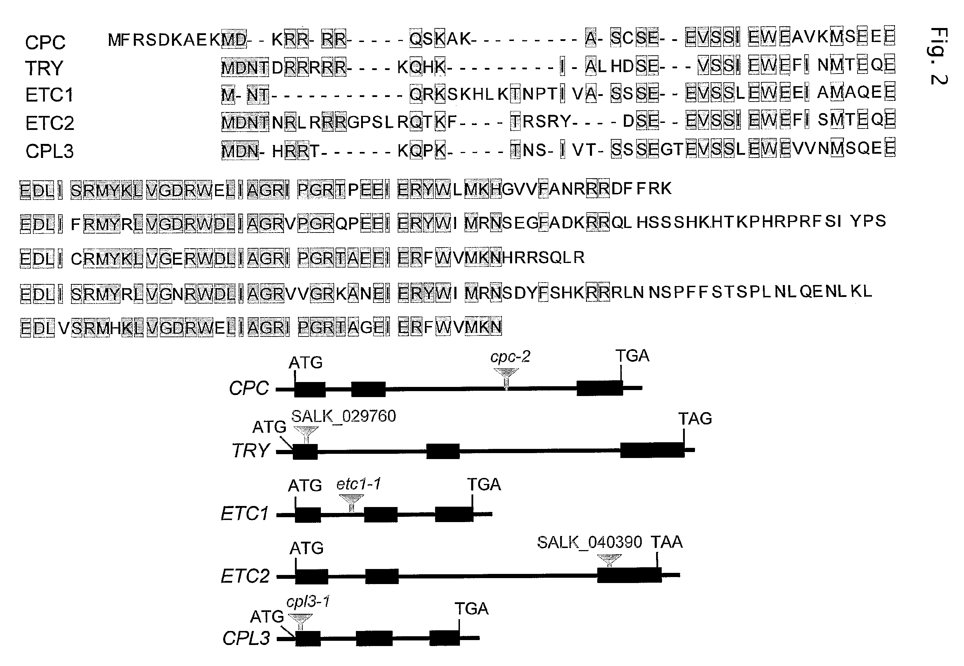

Further, in this Example, a try-29760 mutant in which the TRY gene had been mutated, an etc1-1 mutant in which the ETC1 gene had been mutated, and an etc2-40390 mutant in which the ETC2 gene had been mutated were isolated from the SALK T-DNA group. In addition, a cpc-2 mutant in which the CPC gene had been mutated (disclosed in Kurata, T et al. Cell-to-cell movement of the CAPRICE protein in Arabidopsis root epidermal cell differentiation. Development 132, 5387-5398 (2005)) was prepared. Fig. 2 shows T-DNA insertion sites in the obtained mutants.

### (Experimental example 2) Transformed plant preparation

In this Example, the following transformants were prepared: transformants constitutively expressing the CPC gene, the ETC1 gene, the ETC2 gene, the TRY gene, and the CPL3 gene under the control of the 35S promoter; transformants highly expressing GFP fusion proteins, each of which comprises the respective gene linked to the GFP gene; and transformants expressing the GUS protein, each of which comprises the GUS gene linked to the promoter sequence of the respective gene.

Names, nucleotide sequences, and SEQ ID NOs of promoters used in this Example are listed in table 1.

**Table 1**

| Primer name | Sequence (5' →3' ) | SEQ ID NO |
|---|---|---|
| | | |
| TW1165 | ATATGGTACCAATAAAAAATAAATCAC | SEQ ID NO: 5 |
| TW1166 | TGCTTGTCGACTGTATACACTAA | SEQ ID NO: 6 |
| TW1167 | ATATGGTACCTTTAACATAGAAACCGAC | SEQ ID NO: 7 |
| TW1168 | ATATGTCGACATCTACGACTTAGCTTC | SEQ ID NO: 8 |
| TW1169 | ATATGGTACCACTTCATGTTCTTCCCTT | SEQ ID NO: 9 |
| TW1170 | ATATGTCGACAAGCCAATACATATCCA | SEQ ID NO: 10 |
| RT46 | GGCCAGTCGACAGAAAACTCACTCACTATTCACATC | SEQ ID NO: 11 |
| RT47 | CGAGGATCCACGCTGCGTATTCATCTCAA | SEQ ID NO: 12 |
| RT48 | GGCCAGTCGACGCTTGGCTAGCTCATAAACG | SEQ ID NO: 13 |
| RT49 | CGATCTAGAACGGTTGGTATTATCCATAACTACT | SEQ ID NO: 14 |
| RT50 | GGCCAGTCGACCAGCCCTGAAAACAGCTAAGAA | SEQ ID NO: 15 |
| RT51 | CGAGGATCCGCGATGGTTATCCATGTCAAAC | SEQ ID NO: 16 |
| RT67 | ATATGTCGACAGAAAACTCACTCACTATTCACATC | SEQ ID NO: 17 |
| RT68 | ATATCCCGGGACGTAATTGAGATCTTCGATGATTC | SEQ ID NO: 18 |
| RT69 | ATATGTCGACGCTAGCTCATAAACGTTGGTACG | SEQ ID NO: 19 |
| RT70 | ATATGATATCCAATTTTAGATTTTCTTGGAGATTAAG | SEQ ID NO: 20 |
| RT71 | ATATGTCGACCAGCCCTGAAAACAGCTAAGAA | SEQ ID NO: 21 |
| RT72 | ATATGATATCATTTTTCATGACCCAAAACCTCT | SEQ ID NO: 22 |
| RT73 | CGATGGATAACACTGACCGTCGTC | SEQ ID NO: 23 |
| RT88 | ATATGGATCCACGGTCAGTGTTATCCATTACTATT | SEQ ID NO: 24 |
| RT89 | ATATGTCGACCTCAATATATCAAATTCAAACATTCA | SEQ ID NO: 25 |
| RT90 | ATATCCCGGGGGAAGGATAGATAGAAAAGCGAG | SEQ ID NO: 26 |
| RT91 | ATATGGATCCGTTGGACATTTCCTTCTCTCTC | SEQ ID NO: 27 |
| RT92 | ATATGTCGACCTAACCGCATGGATTAAAGTTG | SEQ ID NO: 28 |
| RT122 | GCGATCGTAAATCTTTGTGTACTAAG | SEQ ID NO: 29 |
| RT123 | CTCAGGAACAAAACTGCAGAATTAC | SEQ ID NO: 30 |
| RT124 | GATAATACCAACCGTCTTCGTCTTC | SEQ ID NO: 31 |
| RT125 | TTCTTGGAGATTAAGAGGAGAAGTAG | SEQ ID NO: 32 |
| RT126 | GATAACCATCGCAGGACTAAGC | SEQ ID NO: 33 |
| RT127 | TACAACGGAATATAATCGAAACAATC | SEQ ID NO: 34 |
| RT128 | CTTCTTGTTTCTCGAGATTTATTCTC | SEQ ID NO: 35 |
| RT129 | AATAGTAATTCAAGGACAGGTACATTTC | SEQ ID NO: 36 |

In order to obtain transformants constitutively expressing the respective gene under the control of the 35S promoter, the following constructs were prepared. Specifically, a 0.7-kb PCR fragment containing the ETC1 gene was first amplified using TW1169 and TW1170. Also, a 1.4-kb PCR fragment containing the ETC2 gene was amplified using TW1165 and TW1166. Further, a 0.8-kb PCR fragment containing the CPL3 gene was amplified using TW1167 and TW1168. Furthermore, a 1.4-kb PCR fragment containing the TRY gene was amplified using RT91 and RT92. Next, the obtained PCR fragments were each subcloned into pBluescript SK+ (Stratagene) with the use of Pyrobest DNA polymerase (Takara, Japan). The prepared plasmids were designated as pBS-ETC1, pBS-ETC2, pBS-CPL3, and pBS-TRY.

Next, fragments obtained by treating pBS-ETC1, pBS-ETC2, and pBS-CPL3 with *Acc*65I and *Sal*I were separately inserted into the *Acc*65I and *Sal*I sites of a pCHF3 binary vector (Jarvis, P. et al., An Arabidopsis mutant defective in the plastid general protein import apparatus. Science 282, 100-103 (1998)) such that 35S::ETC1, 35S:: ETC2, and 35S::CPL3 constructs were prepared. In addition, 35S::TRY construct was prepared in the manner described above, except that pBS-TRY was treated with *Bam*HI and *Sal*I.

The following constructs were prepared in order to obtain transformants highly expressing a GFP fusion protein, each of which comprises the respective gene linked to the GFP gene. Specifically, a 2.3-kb PCR fragment containing the ETC1 gene was amplified using RT67 and RT68. The resultant was subjected to restriction enzyme treatment with the use of *Sal*I and *Sma*I. Also, a 4.0-kb PCR fragment containing the ETC2 gene was amplified using RT69 and RT70. The resultant was digested with *Sal*I and *Eco*RV. Further, a 3.0-kb PCR fragment was amplified using RT71 and RT72. The resultant was subjected to restriction enzyme treatment with the use of *Sal*I and *Eco*RV. Furthermore, a 4.0-kb PCR fragment was amplified using RT89 and RT90. The resultant was subjected to restriction enzyme treatment with the use of *Sal*I and *Sma*I. The obtained fragments were separately inserted into the *Sal*I and *Eco*RV sites of pBS-2xGFP (Kurata, T. et al., Cell-to-cell movement of the CAPRICE protein in Arabidopsis root epidermal cell differentiation. Development 132, 5387-5398 (2005)). Accordingly, pBS-ETC1:2xGFP, pBS-ETC2:2xGFP, pBS-CPL3:2xGFP, and pBS-TRY:2xGFP were prepared.

Next, a fragment obtained by treating pBS-ETC1:2xGFP with *Sal*I and *Xba*I was inserted into the *Sal*I and *Xba*I sites of a pJHA212K binary vector (Yoo, S. Y. et al., The 35S promoter used in a selectable marker gene of a plant transformation vector affects the expression of the transgene. Planta 221, 523-530 (2005)). In addition, a fragment obtained by treating pBS-ETC2:2xGFP with *Sal*I and *Sac*II was inserted into the *Sal*I and *Sma*I sites of a pJHA212K binary vector. Further, fragments obtained by treating pBS-CPL3:2xGFP and pBS-TRY:2xGFP with *Sal*I and *Sac*I were separately inserted into the *Sal*I and *Sac*I sites of a pJHA212K binary vector.

The following constructs were prepared in order to obtain transformants expressing the GUS protein, each of which comprises the GUS gene linked to the promoter sequence of the respective gene. Specifically, a 1.9-kb PCR fragment containing the ETC1 gene promoter region was amplified using RT46 and RT47. Also, a 3.0-kb PCR fragment containing the ETC2 gene promoter region was amplified using RT48 and RT49. Further, a 2.4-kb PCR fragment containing the CPL3 gene promoter region was amplified using RT50 and RT51. Furthermore, a 3.0-kb PCR fragment containing the TRY gene promoter region was amplified using RT88 and RT89. The obtained amplification fragments were each treated with *Not*I and *Acc*I and then subcloned into pBS. The prepared plasmids were designated as pBS-ETC1, pBS-ETC2, pBS-CPL3, and pBS-TRY, respectively.

Next, fragments obtained by treating pBS-ETC1 and pBS-CPL3 with *Sal*I and *Bam*HI were separately inserted into the *Sal*I and *Bam*HI sites of a pBI101 binary vector (Clontech Laboratories, Inc., CA, USA). The obtained plasmids were designated as ETC1promoter::GUS and CPL3promoter::GUS. In addition, fragments obtained by treating pBS-ETC2 and pBS-TRY with *Sal*I and *Xba*I were separately inserted into the *Sal*I and *Xba*I sites of a pBI101 binary vector. The obtained plasmids were designated as ETC2promoter::GUS and TRYpromoter::GUS.

Transformation was carried out in accordance with the method disclosed in Kurata, T. et al., The YORE-YORE gene regulates multiple aspects of epidermal cell differentiation in Arabidopsis. Plant J 36, 55-66 (2003) with the use of the resultant 35S::ETC1, 35S:: ETC2, 35S::CPL3, ETC1::ETC1:2xGFP, ETC2::ETC2:2xGFP, CPL3::CPL3:2xGFP, TRY::TRY:2xGFP, ETC1 promoter::GUS, CPL3 promoter::GUS, ETC2 promoter::GUS, and TRY promoter::GUS.

### [Example 1] The phenotype of a mutant lacking the CPL3 gene

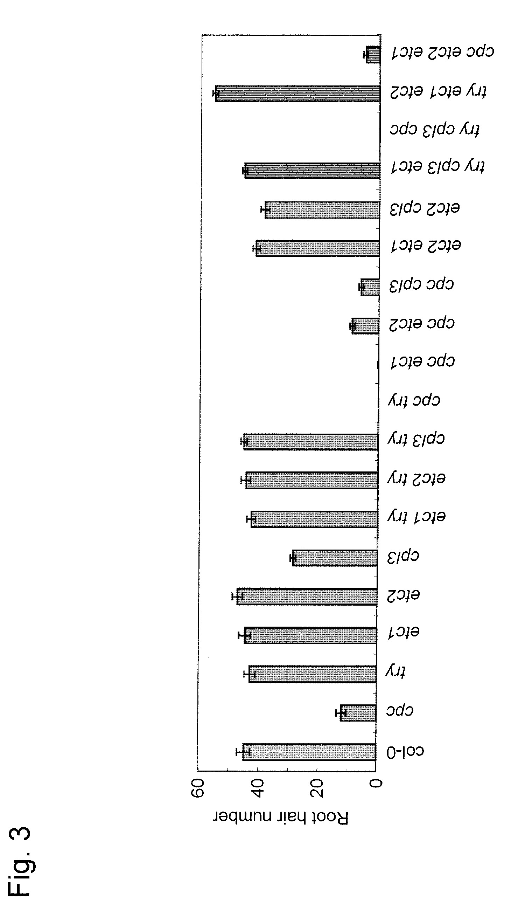

Double mutants and triple mutants with respect to the CPC gene, the TRY gene, the ETC1 gene, the ETC2 gene, and the CPL3 gene were identified by identifying homozygotes of the etc1-1 mutant, the etc2-40390 mutant, and the cpl3-1 mutant isolated in Experimental example 1 in the F2 generation, by PCR. Fig. 3 shows the results of determining the root hair numbers in mutants, the obtained double mutants and triple mutants with respect to the respective genes.

As seen from fig. 3, the root hair number slightly decreased in the cpl3-1 mutant. In addition, the root hair number significantly decreased or substantially no root hair was observed in the double mutant with respect to the CPC gene and the TRY gene, the double mutant with respect to the CPC gene and the ETC1 gene, and the triple mutant with respect to the TRY gene, the CPL3 gene, and the CPC gene.

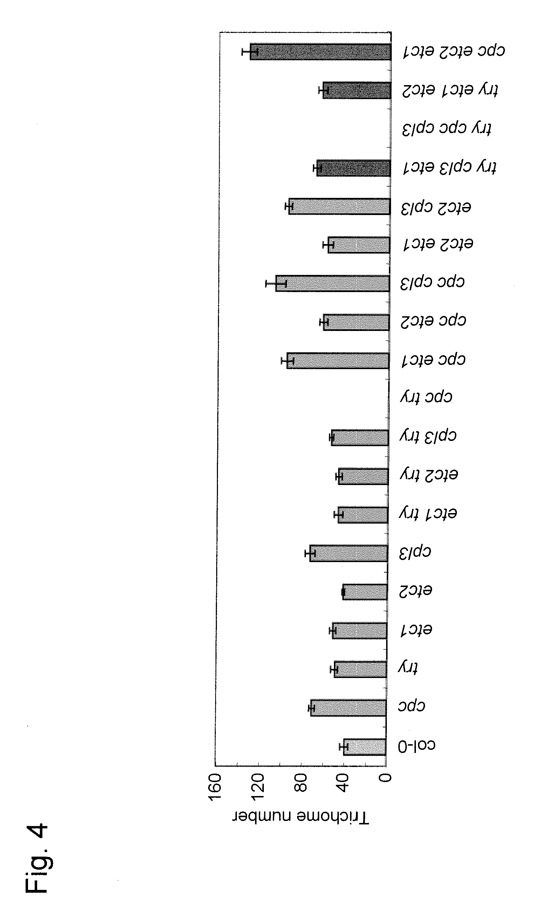

Fig. 4 shows the results of determining the trichome number in mutants, the obtained double mutants and triple mutants, with respect to the respective gene. As seen from fig. 4, the trichome number increased in the cpl3-1 mutant compared with the wild-type plant, as in the case of the cpc-2 mutant. Also, the trichome number further increased in the triple mutant with respect to the TRY gene, the CPC gene, and the CPL3 gene. Further, in the quadruple mutant with respect to the TRY gene, the CPC gene, the ETC1 gene, and the CPL3 gene, a further increased number of trichomes were formed and the entire primary surfaces of leaves were covered with trichomes.

In addition, the cpl3-1 mutant isolated in Experimental example 1 and the transformants prepared in Experimental example 2 were observed in terms of growth.

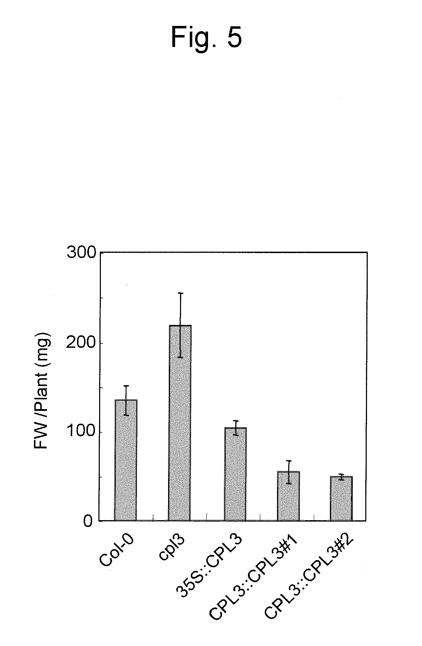

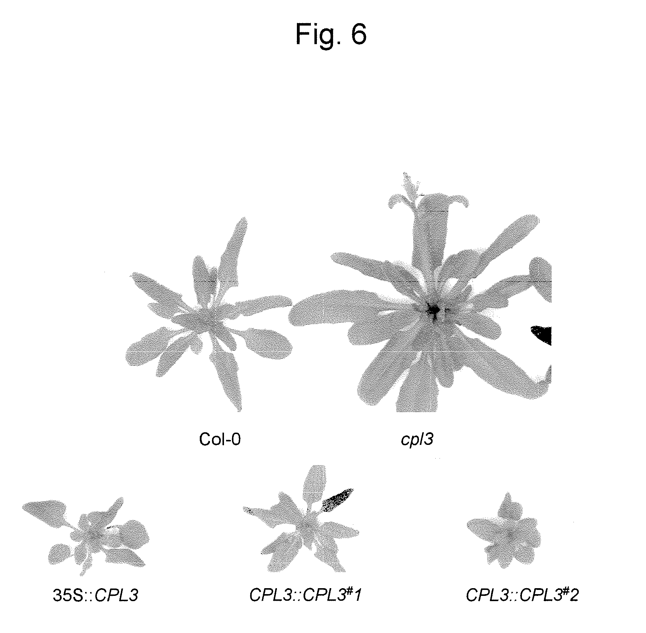

Fig. 5 shows the results of measuring the raw weight in the respective plant at the same growth stage. Fig. 6 shows images of the respective plant at such stage. As seen from figs. 5 and 6, the mutant lacking the CPL3 gene grew to a larger size than the wild-type plant. On the other hand, the growth of transformant overexpressing the CPL3 gene was equivalent to 30% of the growth of the wild-type plant. In addition, the growth of each of the mutants lacking the CPC gene, the ETC 1 gene, the ETC2 gene, or the TRY gene was merely comparable to the growth of the wild-type plant (the results are not shown).

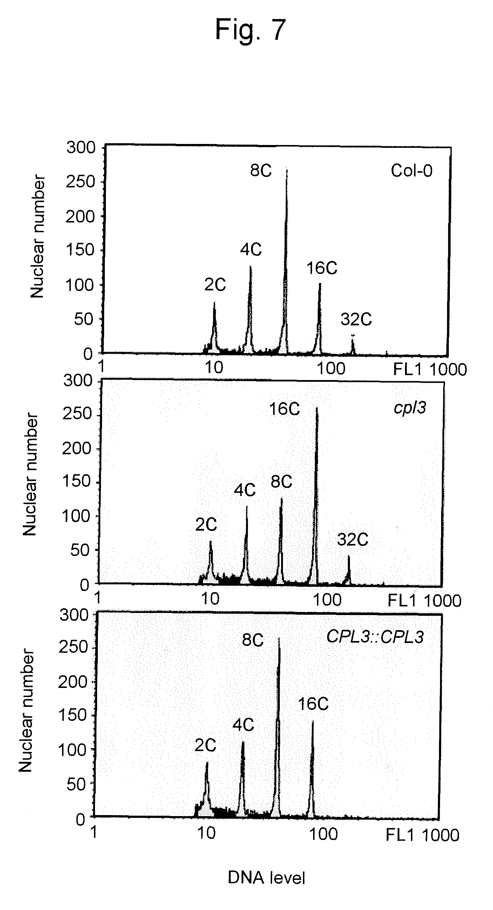

Further, fig. 7 shows the results of polyploidy verification for the respective plant. Incidentally, the respective plant 2 weeks after seeding was subjected to measurement for polyploidy with the use of a Ploidy Analtzer PA Flow Cytometer (Partec) according to the manufacturer' s instructions. As seen from fig. 7, the 16C peak significantly increased in the mutant lacking the CPL3 gene. The results suggest that the mutant lacking the CPL3 gene exhibits a phenotype related to an improvement in cell size due to promoted endoreduplication.

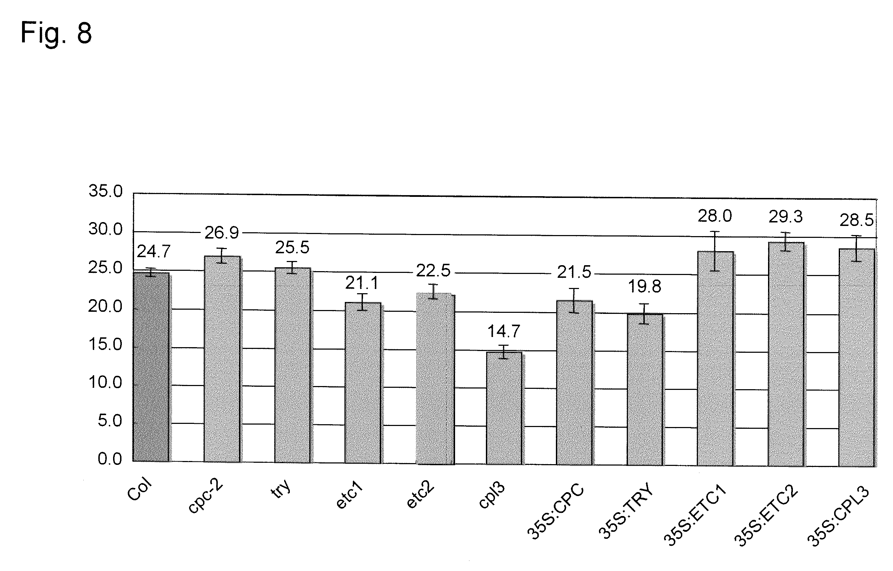

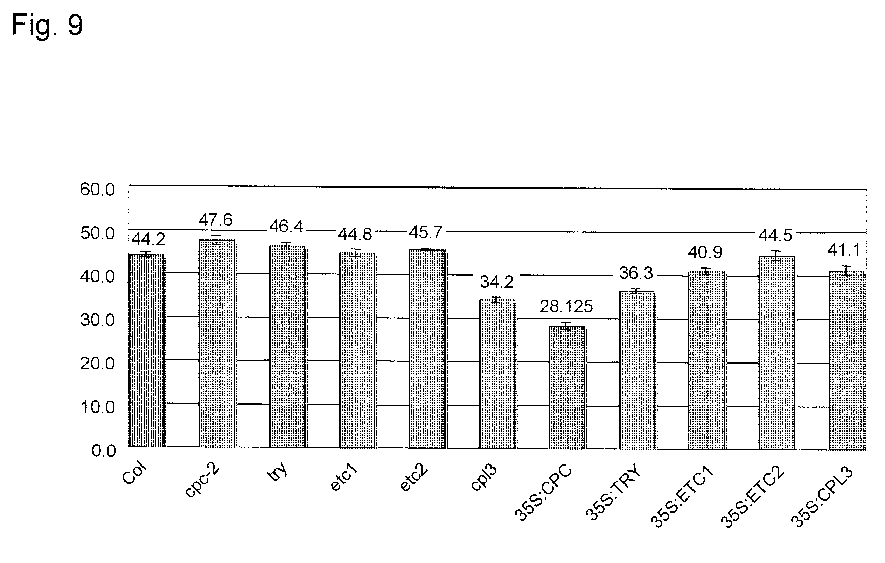

Further, fig. 8 shows the results of determining the true leaf number in the respective plant. Fig. 9 shows results of determining the number of days until bolting of the respective plant. As seen from figs. 8 and 9, the mutant lacking the CPL3 gene flowered earlier than the wild-type plant and the other mutants and transformants.

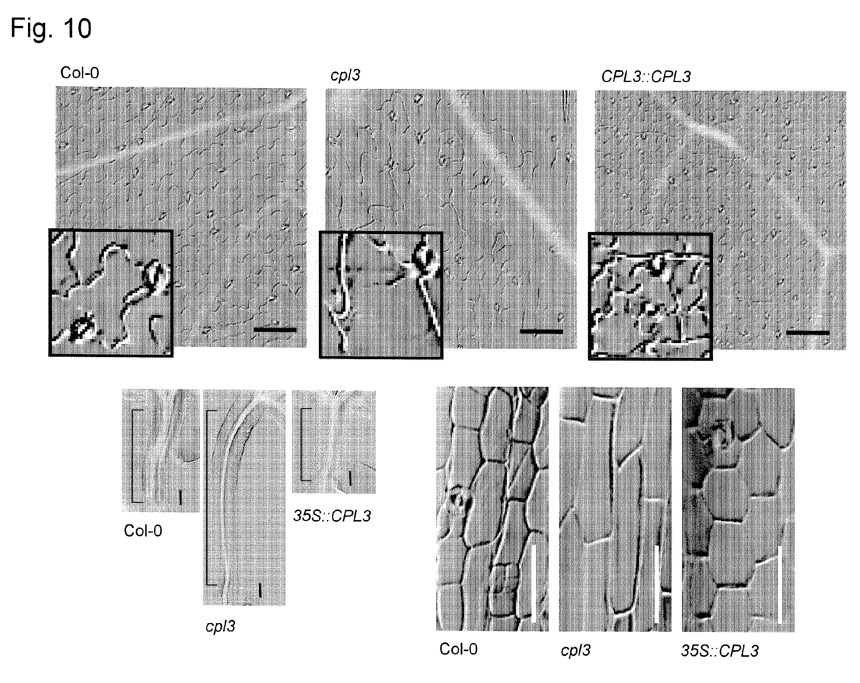

In addition, leaf and hypocotyl cell phenotypes of the respective plants were observed with an optical microscope. Fig. 10 shows the results. As seen from fig. 10, the following features were exhibited by the mutant lacking the CPL3 gene compared with the wild-type plant and transformants overexpressing the CPL3 gene: enlargement in leaf epidermal cells and elongation in hypocotyls. In addition, table 2 lists the results of measuring the leaf size and determining epithelial cell number for the respective plant.

**Table 2**

| Genotype | Leaf size (mm²) | Epithelial cells (number / mm⁻²) | Epithelial cells / leaf |
|---|---|---|---|
| Col-0 | 16.8±0.3 | 181±19 | 3035±311 |
| *cpl3* | 19.0±1.3 | 158±26 | 3014±535 |
| *CPL3::CPL3* | 9.5±0.9 | 311±14 | 2960±306 |

Upon epithelial cell number determination, third leaves were collected 2 weeks after seeding and washed with a washing liquid containing the following components at the following ratio: chloral hydrate (weight) : glycerol (volume) : water (volume) = 8:2:1. Then, visualization was carried out with a Zeiss Axioplan 2 (Carl Zwiss, Germany). In table 2, each value represents the average value of measurement results for five leaves.

Based on the results shown in fig. 10 and table 2, the mutant lacking the CPL3 gene grew to a larger size than the wild-type plant, although the cell number of the mutant did not increase beyond that of the wild-type plant. Thus, it has been revealed that growth at the individual cell level is promoted in the mutant lacking the CPL3 gene to a greater extent than those of the wild-type plant.

### [Example 2]

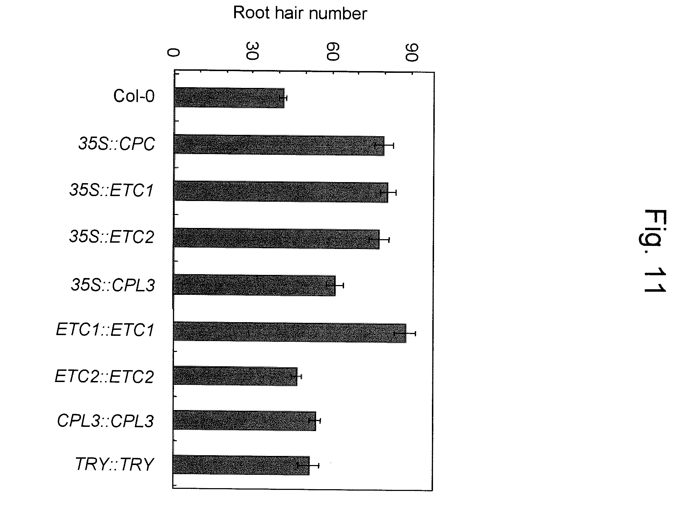

In this Example, transformants overexpressing the CPC gene, the ETC1 gene, the ETC2 gene, the TRY gene, or the CPL3 gene under the control of the 35S promoter or its own promoter were compared with one another in terms of root hair number with the use of the transformants prepared in Experimental example 2. Fig. 11 shows the results. As shown in fig. 11, every transformant constitutively expressing the relevant gene under the 35S promoter exhibited a higher root hair number than the wild-type plant. However, the transformant expressing the CPL3 gene under the control of the 35S promoter exhibited a moderately higher root hair number than the other transformants. Further, the transformant overexpressing the ETC1 gene under the control of the ETC1 promoter exhibited a higher root hair number than the other transformants overexpressing the relevant gene under the control of its own promoter, and the wild-type plant.

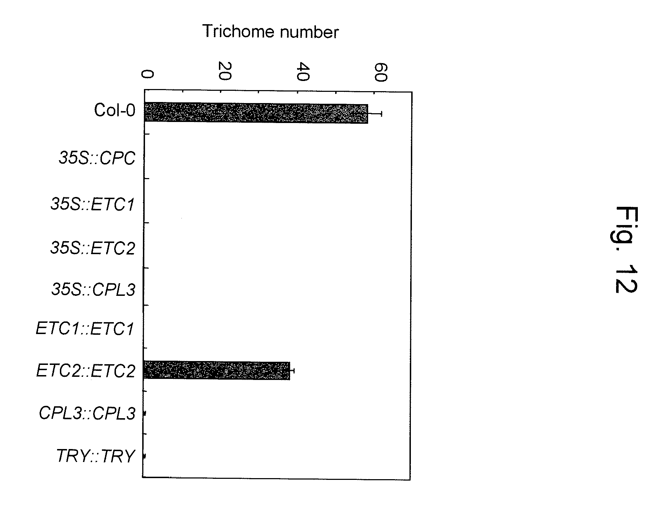

In addition, fig. 12 shows the results of determining the trichome number in the respective transformant in the manner described above. As shown in fig. 12, all transformants, excluding the transformant overexpressing the ETC2 gene under the control of the ETC2 promoter, completely lacked trichomes. Also, the transformant overexpressing the ETC2 gene under the control of the ETC2 promoter had a sharply lower trichome number than the wild-type plant.

### [Example 3]

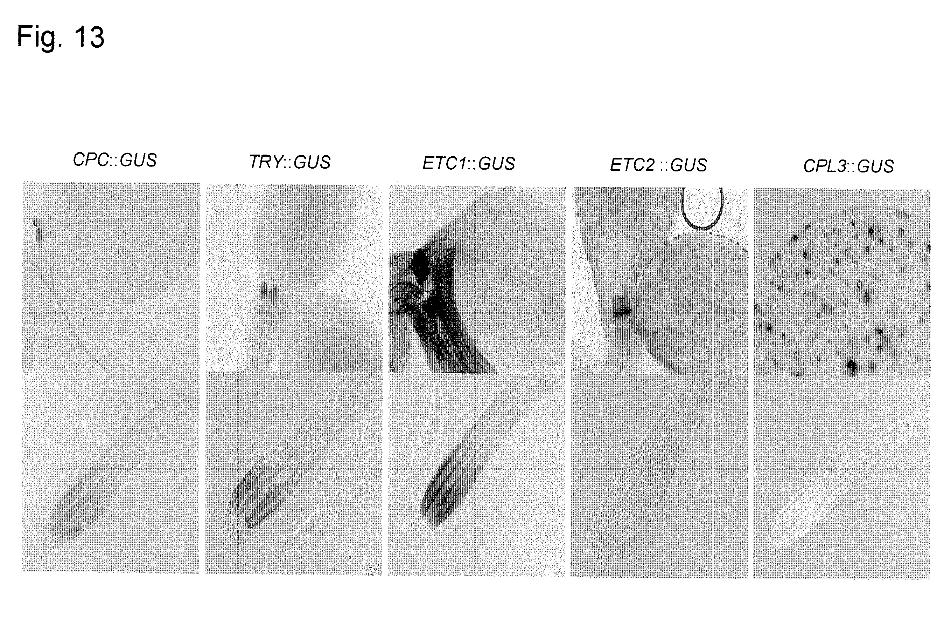

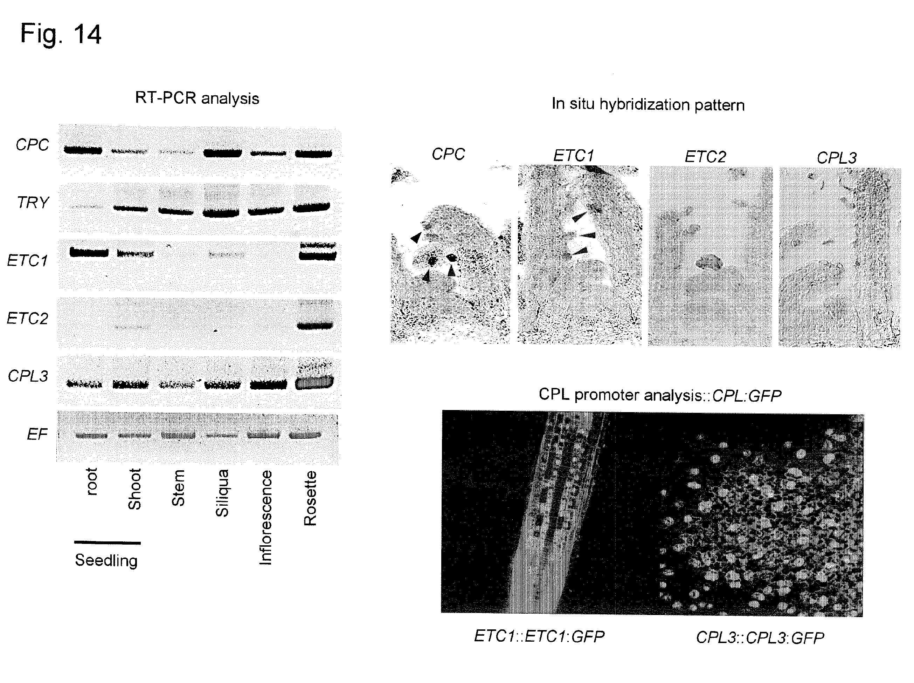

In this Example, the gene expression pattern was examined with the use of the transformants expressing the GUS protein under the control of any promoter of the above genes, which had been prepared in Experimental example 2. Fig. 13 shows the results. In addition, the expression patterns of gene products of the individual genes were examined with the use of the transformants highly expressing the GFP fusion protein (each of which comprises the respective gene), prepared in Experimental example 2. Fig. 14 shows the results. Fig. 14 also shows the results of examining the expression pattern of the respective gene by RT-PCR and *in situ* hybridization.

As shown in figs. 13 and 14, gene products of the CPC gene, TRY gene, and ETC1 gene accumulated specifically in non-root hair cells (hairless cells) of young-leaf trichomes and of root epidermis. In addition, gene products of the ETC2 gene and CPL3 gene accumulated specifically in leaf epidermal cells comprising adaxial-side stomatal guard cells, and not in roots and trichomes. In particular, the CPL3 gene was expressed in a limited manner in stomatal guard cells of leaves, cotyledons, hypocotyls, and petioles, compared to the ETC3 gene.

### [Example 4]

In this Example, cpc, try, etc1, etc2, and cpl3 knockout mutants identified as in the case of Example 1 and a CPL3-overexpressing plant prepared as in the case of Experimental example 2 were examined in terms of time of flowering and leaf number.

Table 3 shows the results of examining the above mutant plants in terms of time of flowering and leaf number at flowering. Incidentally, the data is expressed as the mean ± SD from at least 10 plants for a single experiment.

**Table 3**

| Phenotype | Time of flowering (day) | Leaf number |
|---|---|---|
| Col-0 | 37.6±0.5 | 17.4±0.6 |
| *cpl3* | 28.9±0.5 | 8.2±0.3 |
| *cpc* | 39.6±0.5 | 19.2±0.6 |
| *try* | 36.0±0.8 | 14.3±0.6 |
| *etc1* | 40.6±0.8 | 19.3±0.8 |
| *etc2* | 39.0±0.5 | 14.1±1.9 |
| *35S:CPL3* | 41.1±1.1 | 28.5±1.7 |

As shown in table 3, the cpl3 knockout-mutated plant flowered earlier than the wild-type plant (28.9 ± 0.5 days versus 37.6 ± 0.5 days). In addition, the leaf number thereof was lower than that of the wild-type plant (8.2 ± 0.3 versus 17.4 ± 0.6). On the other hand, there was no significant difference between the wild-type plant and the cpc, try, etc1, or etc2 mutated plant (table 3). The 35S:CPL3 transgenic plant flowered slightly later than the wild-type plant (41.1 ± 1.1 versus 37.6 ± 0.5). In addition, the leaf number thereof was higher than that of the wild-type plant (28.5 ± 1.7 versus 17.4 ± 0.6).

Further, in order to clarify the effects of the CPL3 gene upon flowering, plant lineages with altered CPL3 expression were examined by real-time PCR in terms of the expression of the following genes known to play a central role in the control of floral transition (flowering): FLOWERING LOCUS T (FT), SUPRESSOR OF OVEREXPRESSION OF CO 1 (SOC1), and CONSTANS (CO) (Bagnall, Annals of Botany 71, 75, 1993; Lee et al., Genes & development 14, 2366-2376, 2000; Koornneef et al., Mol Gen Genet 229, 57-66, 1991).

Real-time PCR was conducted as follows. Total RNA was extracted using an RNeasy plant mini kit (Qiagen). On-column DNase I digestion was carried out during RNA purification in accordance with protocols described in the RNeasy mini Handbook. The first-strand cDNA was synthesized from total RNA (1 µg) in a reaction liquid mixture (20 µL) with the use of a Prime Script RT regent kit (Takara). Real-time PCR was performed via the Chromo4 Real-Time PCR Detection System (Bio-Rad, Hercules, CA, USA) with the use of an SYBR Premix Ex Taq (Takara). PCR amplification included a denaturation step at 95°C for 30 seconds, followed by reaction at 95°C for 5 seconds and 60°C for 30 seconds (45 cycles for CPL3 and 40 cycles for CO, FT, SOC1, and ACT2). The relative mRNA levels were calculated with iQ5 software (Bio-Rad) and normalized to the ACT2 mRNA concentration.

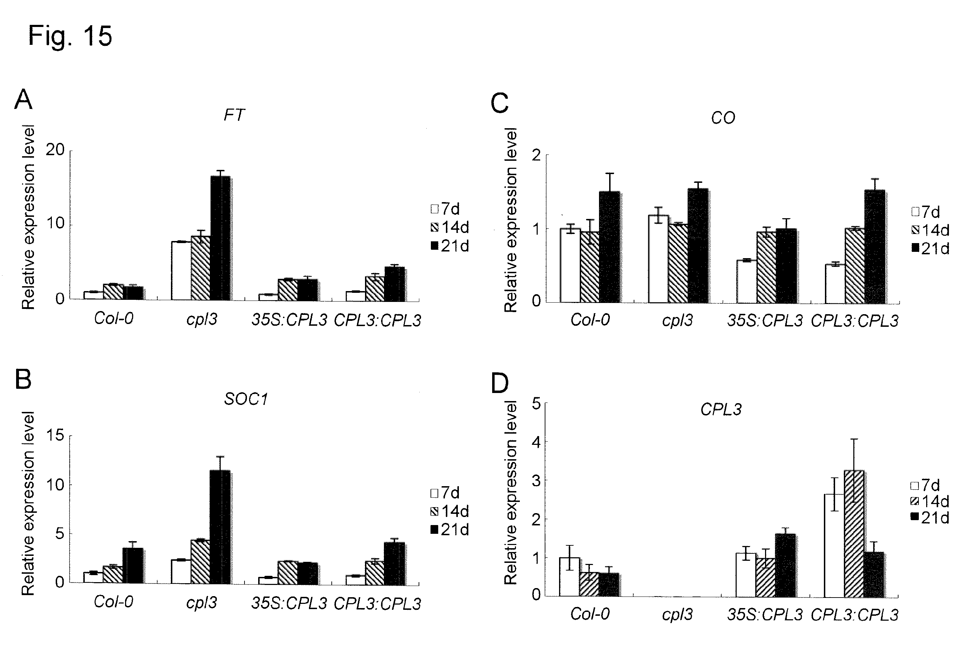

The FT and SOC1 expression levels of the cpl3 knockout mutant were higher than those of the wild-type plant under long day (LD) conditions during leaf growth. The FT expression levels of 21-day-old leaves of the cpl3 knockout mutant were approximately 7.5-fold higher than those of the wild-type plant. The SOC1 expression levels thereof were 3.2-fold higher than the same (figs. 15A and 15B). There was no significant difference between the cpl3 knockout mutant and the wild-type plant in terms of CO expression (fig. 15C). These results indicate that CPL3 is involved in flowering regulation through suppression of FT and SOC 1 expression.

### Industrial Applicability

According to the present invention, it is possible to provide a novel mutated plant that grows to a larger size or flowers earlier than the wild-type plant and a method for production thereof. The mutated plant of the present invention achieves a larger size than that of the wild-type plant. Thus, for instance, the productivity of a substance extracted from a plant can be improved. Also, the mutated plant of the present invention flowers earlier than the wild-type plant and thus fruit or seed formation can be improved. For instance, it is possible to harvest a crop that can generally be harvested just once a year a plurality of times a year. Further, such characteristics of the mutated plant of the present invention result in time and cost saving for crop harvesting.

All publications, patents, and patent applications cited herein are incorporated herein by reference in their entirety.

## Claims

1. A mutated plant in which the function of any one of the following proteins (1) to (3) is suppressed:
(1) a protein comprising the amino acid sequence set forth in SEQ ID NO: 2;
(2) a protein which comprises an amino acid sequence having a substitution. deletion, addition, or insertion of one or more amino acids in the amino acid sequence set forth in SEQ ID NO: 2, and is capable of acting as a transcription factor for controlling epidermal cell differentiation; and
(3) a protein which is encoded by a polynucleotide having a nucleotide sequence with a substitution, deletion, addition, or insertion of one or more nucleotides in the nucleotide sequence set for in SEQ ID NO: 1, and is capable of acting as a transcription factor for controlling epidermal cell differentiation.

2. The mutated plant according to claim 1, wherein the mutated plant lacks a gene encoding any one of the proteins (1) to (3).

3. The mutated plant according to claim 1, wherein the mutated plant is a dicotyledon.

4. The mutated plant according to claim 1, wherein the mutated plant is a plant of the family *Brassicaceae.*

5. A method for producing a mutated plant, comprising suppressing the function of any one of the following proteins (1) to (3) in a target plant:
(1) a protein comprising the amino acid sequence set forth in SEQ ID NO: 2;
(2) a protein which comprises an amino acid sequence having a substitution, deletion, addition, or insertion of one or more amino acids in the amino acid sequence set forth in SEQ ID NO: 2, and is capable of acting as a transcription factor for controlling epidermal cell differentiation; and
(3) a protein which is encoded by a polynucleotide having a nucleotide sequence with a substitution, deletion, addition, or insertion of one or more nucleotides in the nucleotide sequence set forth in SEQ ID NO: 1, and is capable of acting as a transcription factor for controlling epidermal cell differentiation.

6. The method for producing a mutated plant according to claim 5, comprising deleting a gene encoding any one of the proteins (1) to (3).

7. The method for producing a mutated plant according to claim 5, wherein the target plant is a dicotyledon.

8. The method for producing a mutated plant according to claim 5, wherein the target plant is a plant of the family *Brassicaceae.*

9. A method for producing a mutated plant which grows to a larger size or flowers earlier than the wild-type plant, comprising suppressing the function of any one of the following proteins (1) to (3) in a target plant:
(1) a protein comprising the amino acid sequence set forth in SEQ ID NO: 2;
(2) a protein which comprises an amino acid sequence having a substitution, deletion, addition, or insertion of one or more amino acids in the amino acid sequence set forth in SEQ ID NO: 2, and is capable of acting as a transcription factor for controlling epidermal cell differentiation; and
(3) a protein which is encoded by a polynucleotide having a nucleotide sequence with a substitution, deletion, addition, or insertion of one or more nucleotides in the nucleotide sequence set forth in SEQ ID NO: 1, and is capable of acting as a transcription factor for controlling epidermal cell differentiation.

10. The method for producing a mutated plant according to claim 9, comprising deleting a gene encoding any one of the proteins (1) to (3).

11. The method for producing a mutated plant according to claim 9, wherein the target plant is a dicotyledon.

12. The method for producing a mutated plant according to claim 9, wherein the target plant is a plant of the family *Brassicaceae.*


SEQUENCE LISTING

<110> RIKEN

<120> A mutated plant and a method for producing thereof

<130> PH-3281-PCT

<150> JP 2006-278988
<151> 2006-10-12

<160> 36

<170> PatentIn version 3.3

<210> 1
<211> 234
<212> DNA
<213> Arabidopsis thaliana

<220>
<221> CDS
<222> (1)..(234)

<400> 1
atg gat aac cat cgc agg act aag caa ccc aag acc aac tcc atc gtt 48
Met Asp Asn His Arg Arg Thr Lys Gln Pro Lys Thr Asn Ser Ile Val
1               5                   10                  15

act tct tct tct gaa gga aca gaa gtg agt agt ctt gag tgg gaa gtt 96
Thr Ser Ser Ser Glu Gly Thr Glu Val Ser Ser Leu Glu Trp Glu Val
            20                  25                  30

gtg aac atg agt caa gaa gaa gaa gat ttg gtc tct cga atg cat aag 144
Val Asn Met Ser Gln Glu Glu Glu Asp Leu Val Ser Arg Met His Lys
        35                  40                  45

ctt gtc ggt gac agg tgg gaa ctg ata gct ggg agg atc cca gga aga 192
Leu Val Gly Asp Arg Trp Glu Leu Ile Ala Gly Arg Ile Pro Gly Arg
    50                  55                  60

acc gct gga gaa att gag agg ttt tgg gtc atg aaa aat tga 234

Thr Ala Gly Glu Ile Glu Arg Phe Trp Val Met Lys Asn
65                  70                  75

<210>  2
<211>  77
<212>  PRT
<213>  Arabidopsis thaliana

<400>  2

Met Asp Asn His Arg Arg Thr Lys Gln Pro Lys Thr Asn Ser Ile Val
1               5                   10                  15

Thr Ser Ser Ser Glu Gly Thr Glu Val Ser Ser Leu Glu Trp Glu Val
            20                  25                  30

Val Asn Met Ser Gln Glu Glu Glu Asp Leu Val Ser Arg Met His Lys
        35                  40                  45

Leu Val Gly Asp Arg Trp Glu Leu Ile Ala Gly Arg Ile Pro Gly Arg
    50                  55                  60

Thr Ala Gly Glu Ile Glu Arg Phe Trp Val Met Lys Asn
65                  70                  75

<210>  3
<211>  107
<212>  PRT
<213>  Oryza sativa

<400>  3

Met Glu Ser Ser Gly Gly Ser Gln Leu Gly Lys Asn Ser Lys Thr Ser
1               5                   10                  15

Asp Gly Arg Glu Thr Lys Ala Thr Ser Ser Leu Leu Leu Leu Ile Thr
            20                  25                  30

Ala Ile His Phe Val Ala Leu Cys Pro Lys Gly Glu Asn Glu Asp Ala
        35                  40                  45

Arg Lys Glu Val Asn Ser Thr Ala Gln Asn Phe Val His Phe Thr Glu
    50                  55                  60

Glu Glu Glu Asp Ile Val Phe Arg Met His Arg Leu Val Gly Asn Arg
65                  70                  75                  80

Trp Glu Leu Ile Ala Gly Arg Ile Pro Gly Arg Thr Ala Glu Glu Val
                85                  90                  95

Glu Lys Phe Trp Ala Ile Lys His Gln Ala Thr
            100                 105

<210>  4
<211>  83
<212>  PRT
<213>  Oryza sativa

<400>  4

Met Asp Ser Ser Ser Gly Ser Gln Gly Lys Asn Ser Lys Thr Ser Asp
1               5                   10                  15

Gly Cys Glu Thr Lys Glu Val Asn Asn Thr Ala Gln Asn Phe Val His
            20                  25                  30

Phe Thr Glu Glu Glu Glu Asp Leu Val Phe Arg Met His Arg Leu Val
        35                  40                  45

Gly Asn Arg Trp Glu Leu Ile Ala Gly Arg Ile Pro Gly Arg Thr Ala
    50                  55                  60

Lys Glu Gln Tyr Thr Glu Gly Glu Ile Trp Cys Leu Glu Thr Phe Pro
65                  70                  75                  80

Arg Arg Met

<210>  5
<211>  27
<212>  DNA
<213>  Artificial

<220>
<223>  Synthetic DNA

<400>  5
atatggtacc aataaaaat aaatcac                                          27

<210>  6
<211>  23
<212>  DNA
<213>  Artificial

<220>
<223>  Synthetic DNA

<400>  6
tgcttgtcga ctgtatacac taa                                             23

<210>  7
<211>  28
<212>  DNA
<213>  Artificial

<220>
<223>  Synthetic DNA

<400>  7
atatggtacc tttaacatag aaaccgac                                        28

<210> 8
<211> 27
<212> DNA
<213> Artificial

<220>
<223> Synthetic DNA

<400> 8
atatgtcgac atctacgact tagcttc 27

<210> 9
<211> 28
<212> DNA
<213> Artificial

<220>
<223> Synthetic DNA

<400> 9
atatggtacc acttcatgtt cttccctt 28

<210> 10
<211> 27
<212> DNA
<213> Artificial

<220>
<223> Synthetic DNA

<400> 10
atatgtcgac aagccaatac atatcca 27

<210> 11
<211> 36
<212> DNA
<213> Artificial

<220>
<223> Synthetic DNA

<400> 11
ggccagtcga cagaaaactc actcactatt cacatc 36

<210> 12
<211> 29
<212> DNA
<213> Artificial

<220>
<223> Synthetic DNA

<400> 12
cgaggatcca cgctgcgtat tcatctcaa 29

<210> 13
<211> 31
<212> DNA
<213> Artificial

<220>
<223> Synthetic DNA

<400> 13
ggccagtcga cgcttggcta gctcataaac g 31

<210> 14
<211> 34
<212> DNA
<213> Artificial

<220>
<223> Synthetic DNA

<400> 14
cgatctagaa cggttggtat tatccataac tact 34

<210> 15
<211> 33
<212> DNA

<213>  Artificial

<220>
<223>  Synthetic DNA

<400>  15
ggccagtcga ccagccctga aaacagctaa gaa                                    33


<210>  16
<211>  31
<212>  DNA
<213>  Artificial

<220>
<223>  Synthetic DNA

<400>  16
cgaggatccg cgatggttat ccatgtcaaa c                                      31


<210>  17
<211>  35
<212>  DNA
<213>  Artificial

<220>
<223>  Synthetic DNA

<400>  17
atatgtcgac agaaaactca ctcactattc acatc                                  35


<210>  18
<211>  35
<212>  DNA
<213>  Artificial

<220>
<223>  Synthetic DNA

<400>  18
atatcccggg acgtaattga gatcttcgat gattc                                  35

<210> 19
<211> 33
<212> DNA
<213> Artificial

<220>
<223> Synthetic DNA

<400> 19
atatgtcgac gctagctcat aaacgttggt acg 33

<210> 20
<211> 37
<212> DNA
<213> Artificial

<220>
<223> Synthetic DNA

<400> 20
atatgatatc caattttaga ttttcttgga gattaag 37

<210> 21
<211> 32
<212> DNA
<213> Artificial

<220>
<223> Synthetic DNA

<400> 21
atatgtcgac cagccctgaa aacagctaag aa 32

<210> 22
<211> 33
<212> DNA
<213> Artificial

<220>
<223> Synthetic DNA

<400>  22
atatgatatc atttttcatg acccaaaacc tct                                    33

<210>  23
<211>  24
<212>  DNA
<213>  Artificial

<220>
<223>  Synthetic DNA

<400>  23
cgatggataa cactgaccgt cgtc                                              24

<210>  24
<211>  35
<212>  DNA
<213>  Artificial

<220>
<223>  Synthetic DNA

<400>  24
atatggatcc acggtcagtg ttatccatta ctatt                                  35

<210>  25
<211>  36
<212>  DNA
<213>  Artificial

<220>
<223>  Synthetic DNA

<400>  25
atatgtcgac ctcaatatat caaattcaaa cattca                                 36

<210>  26
<211>  33
<212>  DNA

<213>  Artificial

<220>
<223>  Synthetic DNA

<400>  26
atatcccggg ggaaggatag atagaaaagc gag                                    33


<210>  27
<211>  32
<212>  DNA
<213>  Artificial

<220>
<223>  Synthetic DNA

<400>  27
atatggatcc gttggacatt tccttctctc tc                                     32


<210>  28
<211>  32
<212>  DNA
<213>  Artificial

<220>
<223>  Synthetic DNA

<400>  28
atatgtcgac ctaaccgcat ggattaaagt tg                                     32


<210>  29
<211>  26
<212>  DNA
<213>  Artificial

<220>
<223>  Synthetic DNA

<400>  29
gcgatcgtaa atctttgtgt actaag                                            26

<210> 30
<211> 25
<212> DNA
<213> Artificial

<220>
<223> Synthetic DNA

<400> 30
ctcaggaaca aaactgcaga attac 25


<210> 31
<211> 25
<212> DNA
<213> Artificial

<220>
<223> Synthetic DNA

<400> 31
gataatacca accgtcttcg tcttc 25


<210> 32
<211> 26
<212> DNA
<213> Artificial

<220>
<223> Synthetic DNA

<400> 32
ttcttggaga ttaagaggag aagtag 26


<210> 33
<211> 22
<212> DNA
<213> Artificial

<220>
<223> Synthetic DNA

<400> 33
gataaccatc gcaggactaa gc 22

<210> 34
<211> 26
<212> DNA
<213> Artificial

<220>
<223> Synthetic DNA

<400> 34
tacaacggaa tataatcgaa acaatc 26

<210> 35
<211> 26
<212> DNA
<213> Artificial

<220>
<223> Synthetic DNA

<400> 35
cttcttgttt ctcgagattt attctc 26

<210> 36
<211> 28
<212> DNA
<213> Artificial

<220>
<223> Synthetic DNA

<400> 36
aatagtaatt caaggacagg tacatttc 28